(19) Europäisches Patentamt
European Patent Office
Office européen des brevets

(11) EP 4 260 692 A1

(12) **EUROPEAN PATENT APPLICATION**
published in accordance with Art. 153(4) EPC

(43) Date of publication:
**18.10.2023 Bulletin 2023/42**

(21) Application number: **21903313.1**

(22) Date of filing: **03.12.2021**

(51) International Patent Classification (IPC):
***A01K 29/00*** *(2006.01)* ***A61B 5/07*** *(2006.01)*

(52) Cooperative Patent Classification (CPC):
**A01K 29/00; A61B 5/07;** Y02A 40/70

(86) International application number:
**PCT/JP2021/044384**

(87) International publication number:
**WO 2022/124209 (16.06.2022 Gazette 2022/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.12.2020 JP 2020205068**

(71) Applicant: **DAIKIN INDUSTRIES, LTD.**
**Osaka 530-0001 (JP)**

(72) Inventors:
- **ISHII, Kenji**
  **Osaka-Shi, Osaka 530-8323 (JP)**
- **TANAKA, Yoshito**
  **Osaka-Shi, Osaka 530-8323 (JP)**
- **SHIMOSUKI, Takumi**
  **Osaka-Shi, Osaka 530-8323 (JP)**
- **KOMORI, Masaji**
  **Osaka-Shi, Osaka 530-8323 (JP)**
- **MUKAE, Hirofumi**
  **Osaka-Shi, Osaka 530-8323 (JP)**
- **FUKUSHIMA, Toshiyuki**
  **Osaka-Shi, Osaka 530-8323 (JP)**
- **YAMAUCHI, Akiyoshi**
  **Osaka-Shi, Osaka 530-8323 (JP)**
- **KISHIKAWA, Yosuke**
  **Osaka-Shi, Osaka 530-8323 (JP)**
- **AOYAMA, Hirokazu**
  **Osaka-Shi, Osaka 530-8323 (JP)**

(74) Representative: **Hoffmann Eitle**
**Patent- und Rechtsanwälte PartmbB**
**Arabellastraße 30**
**81925 München (DE)**

# (54) SEAL MEMBER FOR LIVESTOCK SENSOR AND LIVESTOCK SENSOR

(57) Provided is a seal for a livestock sensor that has excellent resistance to organic acids and excellently low acid permeability. The disclosure relates to a seal for a livestock sensor containing a resin or a rubber free from a chlorine atom.

FIG. 1

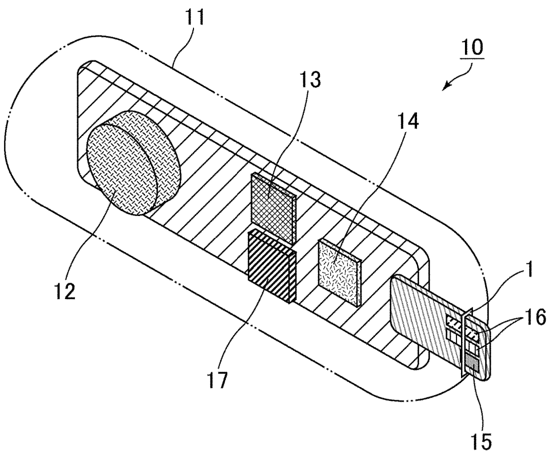




Processed by Luminess, 75001 PARIS (FR)

## Description

TECHNICAL FIELD

The disclosure relates to seals for livestock sensors and livestock sensors.

BACKGROUND ART

In the recent study in the field of livestock production, it has been considered to place sensors in the cattle's body to manage the health and breeding of cattle.

Patent Literature 1 discloses a detection device for detecting an internal state of the first stomach (rumen) of cattle, the detection device including an O-ring for preventing entry of rumen fluid.

CITATION LIST

- Patent Literature

Patent Literature 1: WO 2010/147175

SUMMARY OF INVENTION

- Technical Problem

The disclosure aims to provide a seal for a livestock sensor that has excellent resistance to organic acids and excellently low acid permeability, and a livestock sensor including the same.

- Solution to Problem

The disclosure relates to a seal for a livestock sensor, the seal containing a resin or a rubber free from a chlorine atom.

The resin is preferably a fluororesin.

The fluororesin preferably includes at least one selected from the group consisting of polytetrafluoroethylene, a tetrafluoroethylene/perfluoro(alkyl vinyl ether) copolymer, a tetrafluoroethylene/hexafluoropropylene copolymer, a tetrafluoroethylene/perfluoroalkyl allyl ether copolymer, and a chlorotrifluoroethylene/tetrafluoroethylene/perfluoro(alkyl vinyl ether) copolymer.

The rubber is preferably a fluorine-containing rubber.

The fluorine-containing rubber is preferably a perfluororubber.

The disclosure also relates to a livestock sensor including the seal for a livestock sensor.

- Advantageous Effects of Invention

The disclosure can provide a seal for a livestock sensor that has excellent resistance to organic acids and excellently low acid permeability, and a livestock sensor including the same.

BRIEF DESCRIPTION OF THE DRAWINGS

FIG. 1 is a cross-sectional view of an exemplary structure of a livestock sensor.
FIG. 2 is a cross-sectional diagram of a test device for inspecting chemical permeability.

DESCRIPTION OF EMBODIMENTS

Livestock sensors to be orally administered to livestock (placed in the livestock body, particularly in the stomach) are required to be resistant to organic acids such as gastric acid.

Conventionally, sufficient study has not been made on the material of a seal used in such a livestock sensor.

As a result of intensive studies, the present inventors found that a seal containing a specific material has excellent resistance to organic acids and excellently low acid permeability and thus can be suitably used as a seal for

a livestock sensor. The seal for a livestock sensor of the disclosure has been thus completed.

The disclosure will be specifically described below.

The seal for a livestock sensor of the disclosure contains a resin or a rubber free from a chlorine atom and thus has excellent resistance to organic acids and excellently low acid permeability. When used in a livestock sensor, the seal is less likely to corrode even when it contacts with gastric juice, for example and is not likely to allow permeation of gastric juice, for example, thereby preventing the gastric juice, for example, from entering the inside of the livestock sensor to avoid corrosion of a component such as an internal substrate.

Examples of the resin include a fluororesin and a silicone resin. A fluororesin is preferred as it has much better resistance to organic acids, much lower acid permeability, and a high specific gravity.

The fluororesin preferably has a melting point of 100°C to 360°C, more preferably 140°C to 350°C, still more preferably 160°C to 340°C.

The melting point is the temperature corresponding to the maximum value on a heat-of-fusion curve obtained by increasing the temperature at a rate of 10°C/min using a differential scanning calorimeter (DSC).

The fluororesin preferably has a specific gravity of 1.8 or higher. The fluororesin having a specific gravity within the above range can increase the specific gravity of the livestock sensor, facilitating placement (submerging) of the sensor in a body fluid such as gastric juice. The specific gravity is more preferably 1.9 or higher, still more preferably 2.0 or higher. The upper limit of the specific gravity is preferably, but is not limited to, 3.0 or lower, more preferably 2.5 or lower.

The specific gravity of the fluororesin is measured in conformity with ASTM D-792.

Examples of the fluororesin include polytetrafluoroethylene (PTFE), a tetrafluoroethylene (TFE)/perfluoro(alkyl vinyl ether) (PAVE) copolymer (PFA), a TFE/hexafluoropropylene (HFP) copolymer (FEP), an ethylene (Et)/TFE copolymer (ETFE), an Et/TFE/HFP copolymer (EFEP), polychlorotrifluoroethylene (PCTFE), a chlorotrifluoroethylene (CTFE)/TFE copolymer, a CTFE/TFE/PAVE copolymer, an Et/CTFE copolymer, polyvinyl fluoride (PVF), polyvinylidene fluoride (PVdF), a vinylidene fluoride (VdF)/TFE copolymer, a VdF/HFP copolymer, a VdF/TFE/HFP copolymer, a VdF/HFP/(meth)acrylic acid copolymer, a VdF/CTFE copolymer, a VdF/pentafluoropropylene copolymer, a VdF/PAVE/TFE copolymer, and a TFE/perfluoroalkyl allyl ether copolymer. The perfluoroalkyl allyl ether is a monomer represented by $CF_2=CFCF_2-O-Rf^4$ (wherein $Rf^4$ is a C1-C5 perfluoroalkyl group) .

The fluororesin is preferably a perhalopolymer as it has much better resistance to organic acids and much lower acid permeability. A perhalopolymer is a polymer in which every carbon atom constituting the main chain of the polymer is coupled with a halogen atom.

The fluororesin preferably includes at least one selected from the group consisting of PTFE, PFA, FEP, a TFE/perfluoroalkyl allyl ether copolymer, and a CTFE/TFE/PAVE copolymer, more preferably at least one selected from the group consisting of PTFE, PFA, and a CTFE/TFE/PAVE copolymer.

The PTFE may be a TFE homopolymer consisting only of a tetrafluoroethylene (TFE) unit, or may be a modified PTFE containing a TFE unit and a modifying monomer unit based on a modifying monomer copolymerizable with TFE.

The modifying monomer may be any monomer copolymerizable with TFE, and examples thereof include a perfluoroolefin such as hexafluoropropylene (HFP); a chlorofluoroolefin such as chlorotrifluoroethylene (CTFE); a hydrogen-containing fluoroolefin such as trifluoroethylene and vinylidene fluoride (VdF); a perfluorovinyl ether; a perfluoroalkyl allyl ether; a (perfluoroalkyl)ethylene; and ethylene. One type or two or more types of modifying monomers may be used.

The perfluorovinyl ether is not limited, and may be, for example, an unsaturated perfluoro compound represented by the following formula (1):

$$CF_2=CF-ORf \qquad (1)$$

wherein Rf is a perfluoroorganic group. The term "perfluoro organic group" herein means an organic group in which all hydrogen atoms bonded to any carbon atom are replaced by fluorine atoms. The perfluoro organic group may contain an ether oxygen.

Examples of the perfluorovinyl ether include a perfluoro(alkyl vinyl ether) (PAVE) represented by the formula (1) wherein Rf is a C1-C10 perfluoroalkyl group. The perfluoroalkyl group preferably has 1 to 5 carbon atoms.

Examples of the perfluoroalkyl group in the PAVE include a perfluoromethyl group, a perfluoroethyl group, a perfluoropropyl group, a perfluorobutyl group, a perfluoropentyl group, and a perfluorohexyl group. Preferred is perfluoro(propyl vinyl ether) (PPVE) wherein the perfluoroalkyl group is a perfluoropropyl group.

Examples of the perfluorovinyl ether further include: those represented by the formula (1) wherein Rf is a C4-C9 perfluoro(alkoxyalkyl) group, those represented by the formula (1) wherein Rf is represented by the following formula:

[Chem. 1]

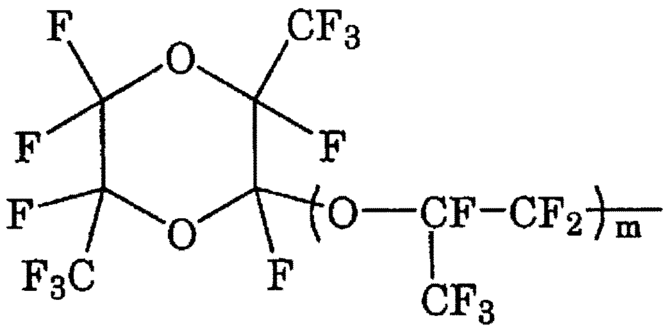

wherein m is 0 or an integer of 1 to 4; and those represented by the formula (1) wherein Rf is a group represented by the following formula:

[Chem. 2]

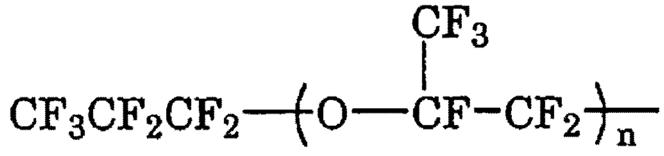

wherein n is an integer of 1 to 4.

Examples of the (perfluoroalkyl)ethylene include, but are not limited to, (perfluorobutyl)ethylene (PFBE), (perfluorohexyl)ethylene (PFHE), and (perfluorooctyl)ethylene.

The modifying monomer in the modified PTFE preferably includes at least one selected from the group consisting of HFP, CTFE, VdF, PPVE, PFBE, and ethylene, more preferably at least one selected from the group consisting of HFP and CTFE.

In the modified PTFE, the amount of the modifying monomer unit is preferably in the range of 0.00001 to 1.0% by mass. The lower limit of the amount of the modifying monomer unit is more preferably 0.0001% by mass, still more preferably 0.001% by mass, even more preferably 0.005% by mass, further preferably 0.010% by mass, particularly preferably 0.030% by mass. The upper limit of the amount of the modifying monomer unit is preferably 0.90% by mass, more preferably 0.50% by mass, still more preferably 0.40% by mass, even more preferably 0.30% by mass.

The term "modifying monomer unit" herein means a moiety that is part of the molecular structure of the modified PTFE and is derived from a modifying monomer. The term "all monomer units" herein means all moieties derived from monomers in the molecular structure of the modified PTFE.

The PTFE preferably has a melting point of 324°C to 360°C. The melting point of PTFE means the first melting point. The first melting point is the temperature corresponding to the maximum value on a heat-of-fusion curve obtained by heating a PTFE that has no history of being heated up to a temperature of 300°C or higher, at a rate of 10°C/min using a differential scanning calorimeter (DSC).

The PTFE preferably has a standard specific gravity (SSG) of 2.130 to 2.280. The standard specific gravity is more preferably 2.220 or lower, still more preferably 2.200 or lower, while it is preferably 2.140 or higher, more preferably 2.150 or higher. The SSG is measured by a water displacement method in conformity with ASTM D-792 using a sample molded in conformity with ASTM D 4895-89.

The PTFE preferably has non-melt secondary processibility. The non-melt secondary processibility means a property of a polymer such that the melt flow rate cannot be measured at a temperature higher than the crystallization melting point in conformity with ASTM D-1238 and D-2116.

The PFA is preferably, but is not limited to, a copolymer having a molar ratio of the TFE unit to the PAVE unit (TFE unit/PAVE unit) of 70/30 or more and less than 99/1, more preferably 70/30 or more and 98.9/1.1 or less, still more preferably 80/20 or more and 98.9/1.1 or less. The PFA is also preferably a copolymer containing 0.1 to 10 mol% (the sum of the TFE unit and the PAVE unit is 90 to 99.9 mol%), more preferably 0.1 to 5 mol%, particularly preferably 0.2 to 4 mol% of a monomer unit derived from a monomer copolymerizable with TFE and PAVE.

Examples of the monomer copolymerizable with TFE and PAVE include HFP, a vinyl monomer represented by the formula (I): $CZ^1Z^2=CZ^3(CF_2)_nZ^4$ (wherein $Z^1$, $Z^2$, and $Z^3$ are the same as or different from each other and each are a hydrogen atom or a fluorine atom; $Z^4$ is a hydrogen atom, a fluorine atom, or a chlorine atom; and n is an integer of 2 to 10), an alkyl perfluorovinyl ether derivative represented by the formula (II): $CF_2=CF-OCH_2-Rf^1$ (wherein $Rf^1$ is a C1-C5 perfluoroalkyl group), and an allyl ether monomer represented by the formula (X): $CZ^5Z^6=CZ^7-CZ^8Z^9-O-Rf^4$

(wherein $Z^5$, $Z^6$, and $Z^7$ are the same as or different from each other and each represent a hydrogen atom, a chlorine atom, or a fluorine atom; $Z^8$ and $Z^9$ each are a hydrogen atom or a fluorine atom; and $Rf^4$ is a C1-C5 perfluoroalkyl group). Examples of the allyl ether monomer include $CH_2$=$CFCF_2$-O-$Rf^4$, $CF_2$=$CFCF_2$-O-$Rf^4$ (perfluoroalkyl allyl ether), $CF_2$=$CFCH_2$-O-$Rf^4$, and $CH_2$=$CHCF_2$-O-$Rf^4$ (in the formulas, $Rf^4$ is the same as that in the formula (X)

Examples of the monomer copolymerizable with TFE and PAVE further include unsaturated monocarboxylic acids, unsaturated dicarboxylic acids, and acid anhydrides of unsaturated dicarboxylic acids, such as itaconic acid, itaconic anhydride, citraconic anhydride, and 5-norbornene-2,3-dicarboxylic anhydride.

The PFA preferably has a melting point of 180°C or higher and lower than 324°C, more preferably 230°C to 320°C, still more preferably 280°C to 320°C.

The FEP is preferably, but is not limited to, a copolymer having a molar ratio of the TFE unit to the HFP unit (TFE unit/HFP unit) of 70/30 or more and less than 99/1, more preferably 70/30 or more and 98.9/1.1 or less, still more preferably 80/20 or more and 98.9/1.1 or less. The FEP is also preferably a copolymer containing 0.1 to 10 mol% (the sum of the TFE unit and the HFP unit is 90 to 99.9 mol%), more preferably 0.1 to 5 mol%, particularly preferably 0.2 to 4 mol% of a monomer unit derived from a monomer copolymerizable with TFE and HFP.

Examples of the monomer copolymerizable with TFE and HFP include PAVE, a monomer represented by the formula (X), and an alkyl perfluorovinyl ether derivative represented by the formula (II).

Examples of the monomer copolymerizable with TFE and HFP further include unsaturated monocarboxylic acids, unsaturated dicarboxylic acids, acid anhydrides of unsaturated dicarboxylic acids, such as itaconic acid, itaconic anhydride, citraconic anhydride, and 5-norbornene-2,3-dicarboxylic anhydride.

The FEP preferably has a melting point of 150°C or higher and lower than 324°C, more preferably 200°C to 320°C, still more preferably 240°C to 320°C.

The CTFE/TFE/PAVE copolymer (CPT) is a copolymer consisting essentially of CTFE, TFE, and PAVE.

The PAVE in the CTFE/TFE/PAVE copolymer may be perfluoro(methyl vinyl ether) (PMVE), perfluoro(ethyl vinyl ether) (PEVE), perfluoro(propyl vinyl ether) (PPVE), perfluoro(butyl vinyl ether), or the like, and preferably includes at least one selected from the group consisting of PMVE, PEVE, and PPVE.

The CTFE/TFE/PAVE copolymer preferably contains the PAVE unit in an amount of 0.5 mol% or more and 5 mol% or less of all monomer units.

The values of constituent units such as a CTFE unit are obtainable by $^{19}$F-NMR analysis.

The CTFE/TFE/PAVE copolymer preferably has a melting point of 160°C to 270°C.

The amounts of the respective monomer units in the polymer described above can be calculated by appropriate combination of NMR, FT-IR, elemental analysis, and X-ray fluorescence analysis in accordance with the types of the monomers.

The fluororesin is also preferably a melt-fabricable fluororesin. Use of a melt-fabricable fluororesin improves processibility.

The term "melt-fabricable" herein means that the polymer can be melted and processed using a conventional processor such as an extruder or an injection molding machine.

The melt-fabricable fluororesin preferably has a melt flow rate (MFR) of 0.1 to 100 g/10 min, more preferably 0.5 to 50 g/10 min.

The MFR herein is a value obtained in conformity with ASTM D1238 using a melt indexer, as the mass (g/10 min) of a polymer flowing out of a nozzle (inner diameter: 2 mm, length: 8 mm) per 10 minutes at a measurement temperature specified according to the type of the fluoropolymer (e.g., 372°C for PFA and FEP, 297°C for CPT and ETFE) and a load specified according to the type of the fluoropolymer (e.g., 5 kg for PFA, FEP, CPT, and ETFE).

Examples of the melt-fabricable fluororesin include those mentioned above, including PFA, FEP, ETFE, EFEP, PCTFE, a CTFE/TFE/PAVE copolymer, and PVdF. The melt-fabricable fluororesin preferably includes at least one selected from the group consisting of PFA, FEP, and a CTFE/TFE/PAVE copolymer.

The rubber free from a chlorine atom is preferably amorphous. The term "amorphous" means that the rubber has a melting peak (ΔH) of 2.0 J/g or lower determined by DSC (temperature-increasing rate: 10°C/min).

Examples of the rubber free from a chlorine atom include fluorine-containing rubbers (excluding those containing chlorine atoms) such as a fluororubber and a fluorosilicone rubber, fluorine-free rubbers free from a chlorine atom.

Examples of the fluororubber include a partially fluorinated rubber and a perfluororubber. A perfluororubber as it has much better resistance to organic acids and much lower acid permeability is preferred.

Examples of the partially fluorinated rubber include a vinylidene fluoride (VdF)-based fluororubber, a tetrafluoroethylene (TFE)/propylene (Pr)-based fluororubber, a tetrafluoroethylene (TFE)/propylene/vinylidene fluoride (VdF)-based fluororubber, an ethylene/hexafluoropropylene (HFP)-based fluororubber, an ethylene/hexafluoropropylene (HFP)/vinylidene fluoride (VdF)-based fluororubber, and an ethylene/hexafluoropropylene (HFP)/tetrafluoroethylene (TFE)-based fluororubber. Preferred among these is at least one selected from the group consisting of a vinylidene fluoride-based fluororubber and a tetrafluoroethylene/propylene-based fluororubber.

The vinylidene fluoride-based fluororubber is preferably a copolymer containing 45 to 85 mol% of vinylidene

fluoride and 55 to 15 mol% of at least one different monomer copolymerizable with vinylidene fluoride. Preferred is a copolymer containing 50 to 80 mol% of vinylidene fluoride and 50 to 20 mol% of at least one different monomer copolymerizable with vinylidene fluoride.

The amounts of the respective monomers constituting the fluoropolymer herein can be calculated by appropriate combination of NMR, FT-IR, elemental analysis, and X-ray fluorescence analysis in accordance with the types of the monomers.

Examples of the at least one different monomer copolymerizable with vinylidene fluoride includes tetrafluoroethylene (TFE), hexafluoropropylene (HFP), a fluoroalkyl vinyl ether, trifluoroethylene, trifluoropropylene, pentafluoropropylene, trifluorobutene, tetrafluoroisobutene, hexafluoroisobutene, vinyl fluoride, a perfluoroalkyl allyl ether, a fluoromonomer represented by the formula (6): $CH_2=CFRf^{61}$ (wherein $Rf^{61}$ is a C1-C12 linear or branched fluoroalkyl group), a fluoromonomer represented by the formula (7): $CH_2=CH-(CF_2)n-X^2$ (wherein $X^2$ is H or F and n is an integer of 3 to 10), a monomer giving a crosslinking site; and non-fluorinated monomers such as ethylene, propylene, and an alkyl vinyl ether. Each of these can be used alone or in any combination. Among these, at least one selected from the group consisting of TFE, HFP, and a fluoroalkyl vinyl ether is preferably used.

The fluoroalkyl vinyl ether preferably includes at least one selected from the group consisting of a fluoromonomer represented by the formula (8):

$$CF_2=CF-ORf^{81}$$

(wherein $Rf^{81}$ is a C1-C8 perfluoroalkyl group), a fluoromonomer represented by the formula (9):

$$CF_2=CFOCF_2ORf^{91}$$

(wherein $Rf^{91}$ is a C1-C6 linear or branched perfluoroalkyl group, a C5-C6 cyclic perfluoroalkyl group, or a C2-C6 linear or branched perfluorooxyalkyl group containing 1 to 3 oxygen atom(s)), and a fluoromonomer represented by the formula (10):

$$CF_2=CFO(CF_2CF(Y^{10})O)_m(CF_2)_nF$$

(wherein $Y^{10}$ is a fluorine atom or a trifluoromethyl group, m is an integer of 1 to 4, and n is an integer of 1 to 4). More preferred is a fluoromonomer represented by the formula (8).

The perfluoroalkyl allyl ether is preferably a fluoromonomer represented by the formula (11): $CF_2=CF-CF_2ORf^{111}$ (wherein $Rf^{111}$ is a C1-C5 perfluoroalkyl group).

Specific examples of the vinylidene fluoride-based fluororubber include a VdF/HFP-based rubber, a VdF/HFP/TFE-based rubber, a rubber based on VdF and a fluoromonomer rubber represented by the formula (6), a rubber based on VdF, a fluoromonomer represented by the formula (6), and TFE, a VdF/perfluoro(methyl vinyl ether) (PMVE)-based rubber, a VdF/PMVE/TFE-based rubber, and a VdF/PMVE/TFE/HFP-based rubber. The rubber based on VdF and a fluoromonomer represented by the formula (6) is preferably VdF/$CH_2=CFCF_3$ rubber. The rubber based on VdF, a fluoromonomer represented by the formula (6), and TFE is preferably VdF/TFE/$CH_2=CFCF_3$ rubber.

The VdF/$CH_2=CFCF_3$-based rubber is preferably a copolymer containing 40 to 99.5 mol% of VdF and 0.5 to 60 mol% of $CH_2=CFCF_3$, more preferably a copolymer containing 50 to 85 mol% of VdF and 20 to 50 mol% of $CH_2=CFCF_3$.

The tetrafluoroethylene/propylene-based fluororubber is preferably a copolymer containing 45 to 70 mol% of tetrafluoroethylene, 55 to 30 mol% of propylene, and 0 to 5 mol% of a fluoromonomer giving a crosslinking site.

The perfluororubber preferably includes at least one selected from the group consisting of perfluororubbers containing TFE, such as a copolymer of TFE/a fluoromonomer represented by the formula (8), (9), or (10) and a copolymer of TFE/a fluoromonomer represented by the formula (8), (9), or (10)/a monomer giving a crosslinking site.

In the case of a TFE/PMVE copolymer, the compositional ratio is preferably (45 to 90)/(10 to 55) (mol%), more preferably (55 to 80)/(20 to 45), still more preferably (55 to 70)/(30 to 45).

In the case of a copolymer of TFE/PMVE/a monomer giving a crosslinking site, the compositional ratio is preferably (45 to 89.9)/(10 to 54.9)/(0.01 to 4) (mol%), more preferably (55 to 77. 9)/(20 to 49.9)/(0.1 to 3.5), still more preferably (55 to 69.8)/(30 to 44.8)/(0.2 to 3).

In the case of a copolymer of TFE/a C4-C12 fluoromonomer represented by the formula (8), (9), or (10), the compositional ratio is preferably (50 to 90)/(10 to 50) (mol%), more preferably (60 to 88)/(12 to 40), still more preferably (65 to 85)/(15 to 35).

In the case of a copolymer of TFE/a C4-C12 fluoromonomer represented by the formula (8), (9), or (10)/a monomer giving a crosslinking site, the compositional ratio is preferably (50 to 89.9)/(10 to 49.9)/(0.01 to 4) (mol%), more preferably (60 to 87.9)/(12 to 39.9)/(0.1 to 3.5), still more preferably (65 to 84.8)/(15 to 34.8)/(0.2 to 3).

If the compositional ratios are out of the ranges mentioned above, the copolymers tend to lose the properties

as a rubber elastic body and to have the properties similar to those of resin.

The perfluororubber preferably includes at least one selected from the group consisting of a copolymer of TFE/a fluoromonomer represented by the formula (10)/a fluoromonomer giving a crosslinking site, a copolymer of TFE/a perfluorovinyl ether represented by the formula (10), a copolymer of TFE/a fluoromonomer represented by the formula (8), and a copolymer of TFE/a fluoromonomer represented by the formula (8)/a monomer giving a crosslinking site.

Examples of the perfluororubber include the perfluororubbers disclosed in WO 97/24381, JP S61-57324 B, JP H4-81608 B, and JP H5-13961 B.

The monomer giving a crosslinking site is a monomer (cure-site monomer) containing a crosslinkable group that can give a fluoropolymer a crosslinking site to form a crosslink with use of a crosslinking agent.

The monomer giving a crosslinking site preferably includes at least one selected from the group consisting of:

a fluoromonomer represented by the formula (12):

$$CX^3_2=CX^3-R_f^{121}CHR^{121}X^4$$

(wherein $X^3$ is a hydrogen atom, a fluorine atom, or $CH_3$; $R_f^{121}$ is a fluoroalkylene group, a perfluoroalkylene group, a fluoro(poly)oxyalkylene group, or a perfluoro(poly)oxyalkylene group; $R^{121}$ is a hydrogen atom or $CH_3$; and $X^4$ is an iodine atom or a bromine atom);
a fluoromonomer represented by the formula (13):

$$CX^3_2=CX^3-R_f^{131}X^4$$

(wherein $X^3$ is a hydrogen atom, a fluorine atom, or $CH_3$; $R_f^{131}$ is a fluoroalkylene group, a perfluoroalkylene group, a fluoropolyoxyalkylene group, or a perfluoropolyoxyalkylene group; and $X^4$ is an iodine atom or a bromine atom);
a fluoromonomer represented by the formula (14):

$$CF_2=CFO(CF_2CF(CF_3)O)_m(CF_2)_n-X^5$$

(wherein m is an integer of 0 to 5; n is an integer of 1 to 3; and $X^5$ is a cyano group, a carboxy group, an alkoxycarbonyl group, an iodine atom, a bromine atom, or-$CH_2I$);
a fluoromonomer represented by the formula (15):

$$CH_2=CFCF_2O(CF(CF_3)CF_2O)_m(CF(CF_3))_n-X^6$$

(wherein m is an integer of 0 to 5; n is an integer of 1 to 3; $X^6$ is a cyano group, a carboxy group, an alkoxycarbonyl group, an iodine atom, a bromine atom, or $-CH_2OH$), and
a monomer represented by the formula (16):

$$CR^{162}R^{163}=CR^{164}-Z-CR^{165}=CR^{166}R^{167}$$

(wherein $R^{162}$, $R^{163}$, $R^{164}$, $R^{165}$, $R^{166}$, and $R^{167}$ are the same as or different from each other, and are each a hydrogen atom or a C1-C5 alkyl group; Z is a C1-C18 linear or branched alkylene group optionally containing an oxygen atom, a C3-C18 cycloalkylene group, a C1-C10 alkylene or oxyalkylene group that is at least partially fluorinated, or a (per)fluoropolyoxyalkylene group represented by $-(Q)_p-CF_2O-(CF_2CF_2O)_m(CF_2O)_n-CF_2-(Q)_p-$ (wherein Q is an alkylene group or an oxyalkylene group; p is 0 or 1; and m/n is 0.2 to 5) and having a molecular weight of 500 to 10000).

$X^3$ is preferably a fluorine atom. $R_f^{121}$ and $R_f^{131}$ are preferably C1-C5 perfluoroalkylene groups. $R^{121}$ is preferably a hydrogen atom. $X^5$ is preferably a cyano group, an alkoxycarbonyl group, an iodine atom, a bromine atom, or $-CH_2I$. $X^6$ is preferably a cyano group, an alkoxycarbonyl group, an iodine atom, a bromine atom, or-$CH_2OH$.

The monomer giving a crosslinking site preferably includes at least one selected from the group consisting of $CF_2=CFOCF_2CF(CF_3)OCF_2CF_2CN$, $CF_2=CFOCF_2CF(CF_3)OCF_2CF_2COOH$, $CF_2=CFOCF_2CF(CF_3)OCF_2CF_2CH_2I$, $CF_2=CFOCF_2CF_2CH_2I$, $CH_2=CFCF_2OCF(CF_3)CF_2OCF(CF_3)CN$, $CH_2=CFCF_2OCF(CF_3)CF_2OCF(CF_3)COOH$, $CH_2=CFCF_2OCF(CF_3)CF_2OCF(CF_3)CH_2OH$, $CH_2=CHCF_2CF_2I$, $CH_2=CH(CF_2)_2CH=CH_2$, $CH_2=CH(CF_2)_6CH=CH_2$, and $CF_2=CFO(CF_2)_5CN$, more preferably at least one selected from the group consisting of $CF_2=CFOCF_2CF(CF_3)OCF_2CF_2CN$ and $CF_2=CFOCF_2CF_2CH_2I$.

In order to achieve excellent compression set performance at high temperature, the fluororubber preferably has a glass transition temperature of -70°C or higher, more preferably -60°C or higher, still more preferably -50°C or

higher. In order to achieve good cold resistance, the fluororubber has a glass transition temperature of preferably 5°C or lower, more preferably 0°C or lower, still more preferably -3°C or lower.

The glass transition temperature can be determined as follows. Specifically, using a differential scanning calorimeter (DSC822e available from Mettler-Toledo International Inc.), 10 mg of a sample is heated at a rate of 10°C/min to give a DSC curve, and the temperature is read at the intermediate point of two intersections between each of the extension lines of the base lines before and after the secondary transition of the DSC curve and the tangent line at the inflection point of the DSC curve.

In order to achieve good heat resistance, the fluororubber preferably has a Mooney viscosity ML(1+20) at 170°C of 30 or higher, more preferably 40 or higher, still more preferably 50 or higher. In order to achieve good processibility, the fluororubber preferably has a Mooney viscosity ML(1+20) at 170°C of 150 or lower, more preferably 120 or lower, still more preferably 110 or lower.

In order to achieve good heat resistance, the fluororubber preferably has a Mooney viscosity ML(1+20) at 140°C of 30 or higher, more preferably 40 or higher, still more preferably 50 or higher. In order to achieve good processibility, the fluororubber preferably has a Mooney viscosity ML(1+20) at 140°C of 180 or lower, more preferably 150 or lower, still more preferably 110 or lower.

In order to achieve good heat resistance, the fluororubber preferably has a Mooney viscosity ML(1+10) at 100°C of 10 or higher, more preferably 20 or higher, still more preferably 30 or higher. In order to achieve good processibility, the fluororubber preferably has a Mooney viscosity ML(1+10) at 100°C of 120 or lower, more preferably 100 or lower, still more preferably 80 or lower.

The Mooney viscosity can be determined using a Mooney viscometer MV2000E available from Alpha Technologies Inc. at 170°C, 140°C, or 100°C in conformity with JIS K 6300.

Examples of the fluorine-free rubbers free from a chlorine atom include nitrile rubber, hydrogenated nitrile rubber, styrene-butadiene rubber, polybutadiene rubber, natural rubber, isoprene rubber, ethylene-$\alpha$-olefin rubber, ethylene-$\alpha$-olefin-nonconjugated diene rubber, acrylic rubber, ethylene acrylic rubber, silicone rubber, butyl rubber, ethylene-vinyl ester rubber, and ethylene-methacrylate rubber. Preferred among these are nitrile rubber and silicone rubber.

In order to achieve much better resistance to organic acids and much lower acid permeability as well as a high specific gravity, the rubber free from a chlorine atom is preferably a fluorine-containing rubber, more preferably a fluororubber, still more preferably a perfluororubber.

The seal of the disclosure may further contain a different component in addition to the resin or rubber. Examples of the different component include various additives such as fillers (e.g., carbon black, barium sulfate), acid acceptors, processing aids (e.g., wax), plasticizers, colorants, stabilizers, adhesion aids, release agents, conductivity-imparting agents, thermal-conductivity-imparting agents, surface non-adhesive agents, flexibility-imparting agents, heat resistance improvers, and flame retardants. The amount of the different component is preferably 0 to 50 parts by mass, more preferably 0 to 20 parts by mass, still more preferably 0 to 10 parts by mass per 100 parts by mass of the resin or rubber.

The seal of the disclosure can be produced by molding the resin by a known molding method such as cutting, injection molding, extrusion molding, or compression molding. The seal of the disclosure can also be produced by crosslink-molding a rubber composition containing the rubber and a crosslinking agent (and a crosslinking aid, if necessary) by a known method.

The seal of the disclosure is used in a livestock sensor to prevent entry of a fluid (liquid or gas) or a foreign substance, preferably a fluid, from the outside. The fluid is preferably a liquid, more preferably a body fluid containing an organic acid in livestock, still more preferably livestock gastric juice, particularly preferably livestock rumen fluid.

The seal preferably prevents a fluid or a foreign substance from entering a space that contains a detecting portion or a substrate of the livestock sensor.

The form of the seal is not limited, and can be determined according to the application site. Examples thereof include a packing, an O-ring, and a gasket.

A livestock sensor to which the seal of the disclosure is applied is placed in the livestock body and detects the state of the livestock (e.g., pH, temperature, amount of exercise (acceleration)). The livestock sensor is preferably configured to be orally administered to livestock. Further, the livestock sensor is preferably a wireless transmission sensor capable of wirelessly transmitting acquired data.

The disclosure also relates to a livestock sensor including the above-described seal of the disclosure. When including the seal of the disclosure, the livestock sensor of the disclosure is less likely to deteriorate even when it contacts with gastric juice, for example, and is not likely to allow permeation of gastric juice, for example, thereby preventing the gastric juice, for example, from entering the inside to avoid corrosion of a component such as an internal substrate.

The livestock sensor preferably includes a housing. The housing is preferably a member capable of containing a detecting portion and other necessary parts inside. Also, the housing of the disclosure may be configured such that a portion thereof can be separated (for example, the main body and the cap).

The housing may be formed of any material, including resins such as a fluororesin or a fluorine-free resin; or metals such as stainless steel. The outer surface of a metal housing may be coated with resin.

The housing may have any shape that can contain the detecting portion and other necessary parts inside, such as a tubular shape (e.g., circular tube, square tube), a bottle shape, a bottomed circular tubular shape, or a bottomed square tubular shape. Preferred among these are a tubular shape, a bottle shape, a bottomed circular tubular shape, and a bottomed square tubular shape, and more preferred are a circular tubular shape and a bottomed circular tubular shape.

The livestock sensor preferably includes a detecting portion contained in the housing. Examples of the detecting portion include a pH sensor, a temperature sensor, a piezoelectric sensor, an acceleration sensor, and a position sensor.

The livestock are preferably ruminant animals including cattle (dairy cattle, beef cattle), sheep, and goats. Cattle are particularly preferred.

The livestock sensor is preferably placed in an internal organ of livestock, more preferably in the stomach, still more preferably in the rumen, particularly preferably in the rumen fluid.

The livestock sensor is preferably left in the livestock body for one month or longer, more preferably six months or longer, still more preferably one year or longer, particularly preferably three years or longer.

The livestock sensor preferably has a specific gravity of 1.8 or higher, more preferably 2.0 or higher. The livestock sensor having a specific gravity within the above range can be easily placed (submerged) in a body fluid such as gastric juice.

The livestock sensor may have any size that allows oral administration to livestock. In the case of the circular tubular sensor, the diameter may be 10 to 35 mm and the length may be 40 to 150 mm, for example.

FIG. 1 shows an exemplary structure of the livestock sensor of the disclosure. The livestock sensor of the disclosure is not limited to this.

A livestock sensor 10 in FIG. 1 includes a housing 11.

The housing 11 contains a signal processing circuit 13 connected to a battery 12. The signal processing circuit 13 is provided with an acceleration sensor 14 and a radio transmitter 17. To the signal processing circuit 13 are electrically connected a temperature sensor 15 and a fixed pH sensor 16.

The temperature sensor 15 and the fixed pH sensor 16 are partly exposed outside the housing 11 so as to contact with the rumen fluid.

The housing 11 is provided with a packing 1 to prevent rumen fluid from flowing into the space containing the signal processing circuit 13. The packing 1 corresponds to the seal of the disclosure.

## EXAMPLES

The disclosure will be described in more detail with reference to examples, but the disclosure is not limited only to these examples.

The materials used in the experimental examples are shown below.

PTFE: TFE homopolymer (melting point: 327°C, SSG: 2.2)
PFA: TFE/PPVE copolymer (melting point: 306°C, MFR: 14 g/10 min)
CPT: CTFE/TFE/PPVE copolymer (melting point: 248°C, MFR: 30 g/10 min)
FEP: TFE/HFP copolymer (melting point: 265°C, MFR: 7 g/10 min)
Perfluororubber (FFKM): TFE/PMVE copolymer
Silicone rubber: KE-136Y-U available from Shin-Etsu Chemical Co., Ltd.
Chloroprene rubber (CR): Denka Chloroprene M-40 available from Denka Company Limited
Chlorosulfonated polyethylene rubber (CSM): Hypalon 40 available from DuPont

### Examples 1 to 6 and Comparative Examples 1 and 2

Sheets each having a thickness of 0.2 mm and a diameter of 120 mm were produced from the respective materials by compression molding using a heat press. PTFE was molded at a temperature that is 50°C to 70°C higher than the melting point and at a pressure of 5 MPa. Other resins were each molded at a temperature that is 40°C higher than the melting point and at a pressure of 3 MPa. The rubbers were crosslink-molded by press vulcanization at a temperature of 100°C to 200°C.

Using the resulting sheets, the long-term chemical resistance and the chemical permeability were evaluated by the following methods. Table 1 shows the results.

### <Long-term chemical resistance (organic acid resistance)>

A test piece (the above sheet) was immersed in an 80% aqueous solution of acetic acid at 50°C for one month, and the appearance thereof was observed and evaluated based on the following criteria.

Good: No change in appearance (no change in appearance including discoloration, swelling, or cracks)
Acceptable: Slight change in appearance (slight change in appearance including discoloration, swelling, or cracks)
Poor: Significant change in appearance (significant change in appearance including discoloration, swelling, or cracks)

<Chemical permeability>

A sample sheet 18 (the above sheet) was sandwiched between two glass containers 19a and 19b (each having a capacity of 200 ml) shown in FIG. 2 using O-rings 20. The container 19a on one side of the sheet was charged with 200 ml of hydrochloric acid having a concentration of 35 mass% or nitric acid having a concentration of 60 mass%, and the container 19b on the other side was charged with 200 ml of pure water. The system was placed in a constant-temperature bath (the sample sheet 18 had a wetted surface of 70 mmφ). The system was left in such a state for 40 days, and a sample in an amount of about 1 ml was collected from a sampling port 21 of the container 19b containing pure water. The hydrochloric acid ion concentration or nitric acid ion concentration (Y ppm) of the sample was quantified by ion chromatography (IC7000-E available from Yokogawa Electric Corporation). The permeation amount of hydrochloric acid or nitric acid (X g·cm/cm$^2$) was calculated using the following formula, and the permeation coefficient of hydrochloric acid or nitric acid was determined.

$$X = Y \times 200 \times 0.02 \times 10^{-6}/(3.5 \times 3.5 \times 3.14)$$

[Table 1]

| | | Example 1 | Example 2 | Example 3 | Example 4 | Example 5 | Example 6 | Comparative Example 1 | Comparative Example 2 |
|---|---|---|---|---|---|---|---|---|---|
| Material | | PTFE | PFA | CPT | FFKM | FEP | Silicone rubber | CR | CSM |
| Evaluation item<br>(1) Long-term chemical resistance | | Good | Good | Good | Good | Good | Acceptable | Poor | Poor |
| (2) Chemical permeability ($g \cdot cm/cm^2 \cdot sec$) | 35%HCl | $7.5 \times 10^{-11}$ | $1.5 \times 10^{-11}$ | $0.3 \times 10^{-11}$ | $4.0 \times 10^{-11}$ | $1.4 \times 10^{-11}$ | $> 1.0 \times 10^{-9}$ | $> 1.0 \times 10^{-9}$ | $> 1.0 \times 10^{-9}$ |
| | 60%$HNO_3$ | $5.7 \times 10^{-12}$ | $1.5 \times 10^{-12}$ | $0.4 \times 10^{-12}$ | $50 \times 10^{-12}$ | $1.3 \times 10^{-12}$ | $> 1.0 \times 10^{-10}$ | $> 1.0 \times 10^{-10}$ | $> 1.0 \times 10^{-10}$ |
| Comprehensive evaluation | | Good | Good | Good | Good | Good | Acceptable | Poor | Poor |

REFERENCE SIGNS LIST

1: packing

10: livestock sensor

11: housing

12: battery

13: signal processing circuit

14: acceleration sensor

15: temperature sensor

16: fixed pH sensor

17: radio transmitter

18: sample sheet

19a, 19b: glass container

20: O-ring

21: sampling port

## Claims

1. A seal for a livestock sensor, the seal comprising:

   a resin, or
   a rubber free from a chlorine atom.

2. The seal for a livestock sensor according to claim 1, wherein the resin is a fluororesin.

3. The seal for a livestock sensor according to claim 2, wherein the fluororesin includes at least one selected from the group consisting of polytetrafluoroethylene, a tetrafluoroethylene/perfluoro(alkyl vinyl ether) copolymer, a tetrafluoroethylene/hexafluoropropylene copolymer, a tetrafluoroethylene/perfluoroalkyl allyl ether copolymer, and a chlorotrifluoroethylene/tetrafluoroethylene/perfluoro(alkyl vinyl ether) copolymer.

4. The seal for a livestock sensor according to any one of claims 1 to 3, wherein the rubber is a fluorine-containing rubber.

5. The seal for a livestock sensor according to claim 4, wherein the fluorine-containing rubber is a perfluororubber.

6. A livestock sensor comprising the seal for a livestock sensor according to any one of claims 1 to 5.

FIG. 1

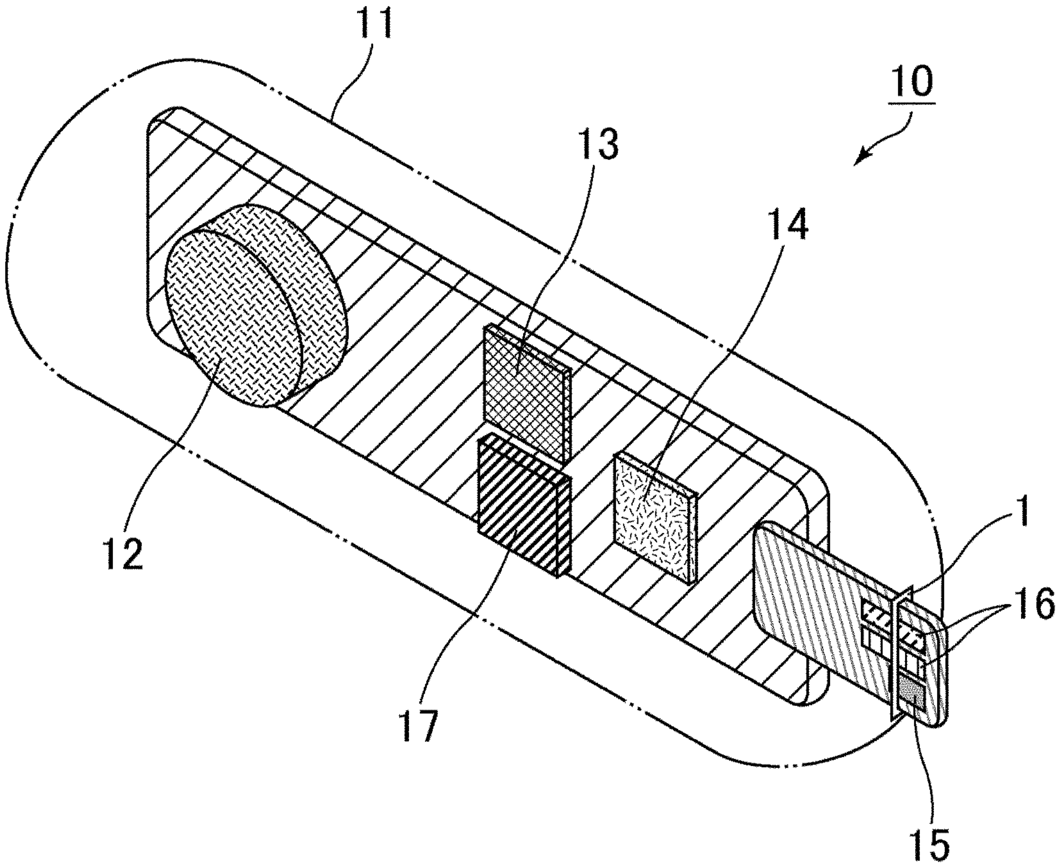

FIG. 2

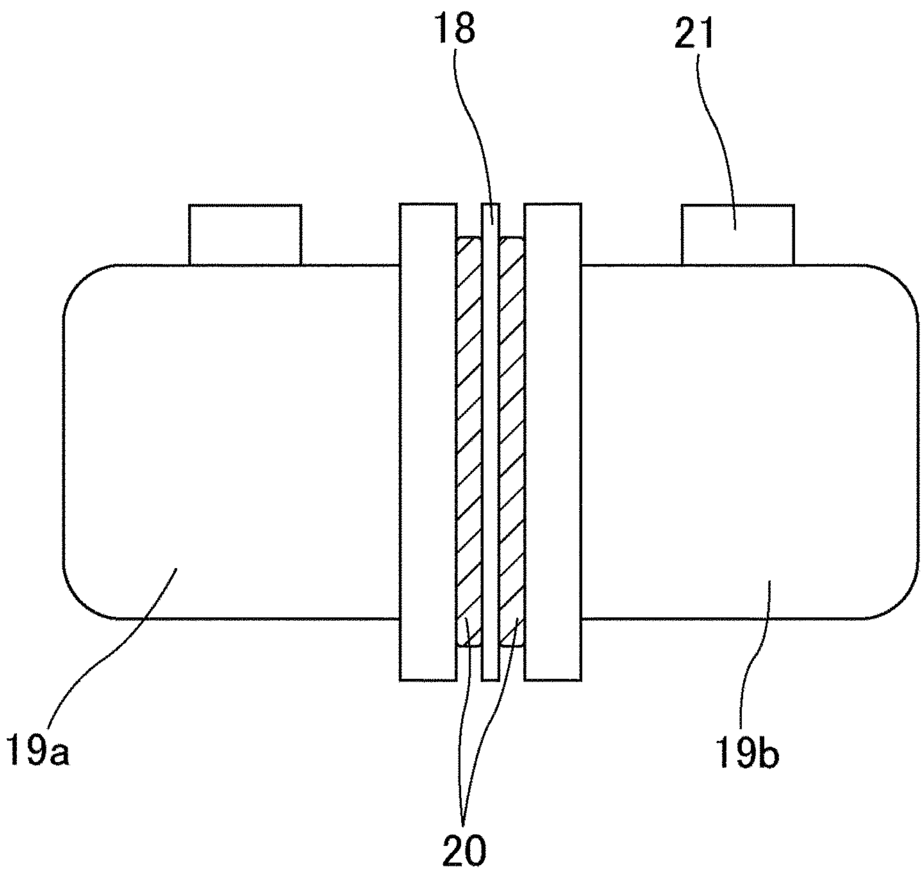

## INTERNATIONAL SEARCH REPORT

International application No.
**PCT/JP2021/044384**

**A. CLASSIFICATION OF SUBJECT MATTER**

***A01K 29/00***(2006.01)i; ***A61B 5/07***(2006.01)i
FI: A01K29/00 D; A61B5/07 100

According to International Patent Classification (IPC) or to both national classification and IPC

**B. FIELDS SEARCHED**

Minimum documentation searched (classification system followed by classification symbols)

A01K29/00; A61B5/07

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2020-127747 A (MARS INCORPORATED) 27 August 2020 (2020-08-27) abstract, claims, paragraphs [0013]-[0018] | 1-6<br>1-6 |
| X | JP 2018-113902 A (NAT AGRICULTURE & FOOD RES ORG) 26 July 2018 (2018-07-26) abstract, claims, paragraphs [0026] | 1-6 |
| Y | WO 2010/147175 A1 (INCORPORATED NATIONAL UNIVERSITY IWATE UNIVERSITY) 23 December 2010 (2010-12-23) paragraphs [0077]-[0078], fig. 4-8 | 1-6 |
| Y | JP 2019-516495 A (METAMODIX, INC.) 20 June 2019 (2019-06-20) paragraphs [0068]-[0069] | 1-6 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 December 2021** | **28 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

**INTERNATIONAL SEARCH REPORT**
**Information on patent family members**

International application No.
**PCT/JP2021/044384**

| Patent document cited in search report | Publication date (day/month/year) | Patent family member(s) | Publication date (day/month/year) |
|---|---|---|---|
| JP 2020-127747 A | 27 August 2020 | US 2017/0252016 A1<br>abstract, claims, paragraphs [0043]-[0048]<br>US 2017/0252017 A1<br>US 2017/0252018 A1<br>WO 2016/042300 A1<br>WO 2016/042301 A1<br>WO 2016/042302 A1 | |
| JP 2018-113902 A | 26 July 2018 | (Family: none) | |
| WO 2010/147175 A1 | 23 December 2010 | US 2012/0088988 A1<br>paragraphs [0102]-[0103], fig. 4-8<br>EP 2438812 A1 | |
| JP 2019-516495 A | 20 June 2019 | US 2017/0333240 A1<br>paragraphs [0113]-[0114]<br>US 2020/0229958 A1<br>WO 2017/201424 A1<br>KR 10-2019-0035618 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010147175 A
- WO 9724381 A
- JP S6157324 B
- JP H481608 B
- JP H513961 B